# EUROPEAN PATENT APPLICATION

(11) **EP 4 837 581 A1**
(43) Date of publication of application: **11.11.2026**
(21) Application number: 25736227.7
(22) Date of filing: 06.01.2025
(51) Int. Cl.: A61K 31/64, A61K 31/4015, A61K 9/00, A61P 17/06, A61K 8/46, A61K 8/49, A61Q 19/00

(54) **COMPOSITION COMPRISING TUBULIN INHIBITOR FOR PREVENTING OR TREATING SKIN DISEASES**

(30) Priority: 05.01.2024 KR 20240001900
(71) Applicant: Cuepeak Bio. Co., Ltd., Daejeon 34141 (KR)
(72) Inventor: HWANG, Joonsung, Daejeon 34141 (KR); KIM, Jeeeune, Daejeon 34141 (KR)
(74) Representative: dompatent
(86) International application number: PCT/KR2025/000261
(87) International publication number: WO 2025/147180

(57) **Abstract**

The present disclosure relates to a pharmaceutical composition, quasi-drug composition, or cosmetic composition comprising a tubulin inhibitor, especially, ABT-751 or TN16, as an active ingredient, for preventing, alleviating, or treating skin diseases. In addition, the present disclosure relates to a cosmetic composition comprising a tubulin inhibitor as an active ingredient for skin moisturization, whitening, wrinkle alleviation, acne relief, skin barrier strengthening, or skin keratinocyte differentiation. The tubulin inhibitor of the present disclosure can be utilized as an active ingredient that simultaneously exhibits activities, such as skin moisturization, whitening, wrinkle alleviation, acne relief, skin barrier strengthening, or skin keratinocyte differentiation, along with the prevention, treatment, or alleviation of skin diseases.

## Description

### [Technical Field]

The present disclosure relates to a pharmaceutical composition including a tubulin inhibitor as an active ingredient for preventing or treating skin diseases.

In addition, the present disclosure relates to a quasi-drug composition including a tubulin inhibitor as an active ingredient for preventing or alleviating skin diseases.

In addition, the present disclosure relates to a cosmetic composition including a tubulin inhibitor as an active ingredient for preventing or alleviating skin diseases.

In addition, the present disclosure relates to a cosmetic composition including a tubulin inhibitor as an active ingredient for skin moisturization, whitening, wrinkle alleviation, acne relief, skin barrier strengthening, or skin keratinocyte differentiation.

### [Background Art]

A major function of the skin serves as a barrier for separating an internal environment from an external environment, and to protect against attacks of foreign substances and minimize the loss of moisture and other essential body ingredients to an external space. Skin tissue includes various cells, including immune cells responsible for the body's inherent immune functions, along with skin cells that form a layered structure consisting of a basal layer, spinous and granular layers, and a cornified layer. The skin tissue maintains skin homeostasis because these various cells achieve balance and harmony and perform their respective roles, and skin diseases correspond to cases where such skin homeostasis may not be maintained.

Cytokines are known as soluble molecules that play an important role in mediating communication between immune cells, and between immune and non-immune cells. The cytokines generally contribute to host defense and immune homeostasis, but abnormal cytokine production causes dysfunctional immune responses and immune-related diseases including autoimmune, rheumatic, allergic, and inflammatory skin diseases. The cytokines have been reported to play an important role in the regulation of skin inflammation. These signals need to be strictly regulated, and failure of the regulation may cause immune system dysfunction, excessive inflammation, and various immune-related pathological phenomena. In this context, negative regulation of cytokine signaling may be an important target for the treatment of skin diseases.

In addition, cell tight junctions are known to play an important role in epidermal barrier function. The cell tight junctions are cell-cell junction structures that exist in endothelium as well as in monolayer and multilayer epitheliums. The cell tight junctions consist of transmembrane proteins, such as claudin family proteins, tight junction-associated marvel proteins (e.g., occludin, junctional adhesion molecules (JAMs)), and tight junction plaque proteins (e.g., zonula occludens (ZO) proteins 1-3, MUPP-1, and cingulin). These cell tight junctions are connected to the actin filament cytoskeleton. The primary function of the cell tight junctions is to seal intercellular pathways to restrict the movement of molecules within the intercellular space, and the cell tight junctions are also involved in cell differentiation, proliferation, cell polarity, and signaling processes.

Psoriasis, one of the inflammatory skin diseases, is known to be primarily caused by an abnormality in the immune system and is considered a type of autoimmune disease that may have systemic effects. As main symptoms, skin cells proliferate faster than normal cells to cause the epidermis to be thickened and white, scale-like keratins to be accumulated, and red, millet-sized rashes occur on the skin, and layers of silvery-white, dandruff-like keratins are accumulated on the rash sites, and rashes gradually grow larger and thus increase the size of the palm in severe cases. (Skin lesions) Red rashes (plaque psoriasis) covered with silvery-white scales are the most common and occur mainly on sites such as the scalp, elbows, knees, and back. (Itching and pain) The affected area may feel itchy or stinging, and is accompanied by pain in severe cases. (Skin cracking and bleeding) As the lesion worsens, the skin cracks and bleeding occurs. (Nail and joint damage) The symptoms may lead to psoriatic arthritis, causing joint pain, swelling, and nail deformities.

### [Disclosure]

### [Technical Problem]

Accordingly, the present inventors confirmed that a tubulin inhibitor, particularly ABT-751 or TN16, inhibited the signaling of inflammatory cytokines (TNF-α, IL-6, IL-17, IL-23) to reduce inflammatory responses and inhibited the expression of Matrix Metalloproteinases (MMP-1, MMP-3, MMP-9) caused by ultraviolet rays (UV) to inhibit collagen degradation and photoaging. In addition, the present inventors confirmed that the tubulin inhibitor effectively inhibited NF-κB signaling to regulate immune responses and inflammatory mechanisms, and increased the expression of tight junction proteins (Claudin-1, ZO-1, Occludin) to strengthen a skin barrier function. In addition, the present inventors confirmed that a whitening effect was exerted by inhibiting melanin production through the inhibition of tyrosinase activity, and then completed the present disclosure.

Therefore, an object of the present disclosure is to provide a pharmaceutical composition and a quasi-drug composition for preventing or treating skin diseases. Another object of the present disclosure is to provide a cosmetic composition for preventing or alleviating skin diseases and a cosmetic composition for skin moisturization, whitening, wrinkle alleviation, acne relief, skin barrier strengthening, or skin keratinocyte differentiation.

### [Technical Solution]

In order to achieve the object, an aspect of the present disclosure provides a pharmaceutical composition including a tubulin inhibitor, particularly ABT-751 or TN16 as an active ingredient for preventing or treating skin diseases.

Another aspect of the present disclosure provides a quasi-drug composition and a cosmetic composition including the tubulin inhibitor as an active ingredient for preventing or alleviating skin diseases.

Yet another aspect of the present disclosure provides a cosmetic composition including the tubulin inhibitor as an active ingredient for skin moisturization, whitening, wrinkle alleviation, acne relief, skin barrier strengthening, or skin keratinocyte differentiation.

### [Advantageous Effects]

According to the present disclosure, the tubulin inhibitor ABT-751 or TN16 inhibits the signaling of inflammatory cytokines (TNF-α, IL-6, IL-17, IL-23) to reduce inflammatory responses and inhibits the expression of Matrix Metalloproteinases (MMP-1, MMP-3, MMP-9) caused by ultraviolet rays (UV) to reduce collagen degradation and photoaging. In addition, the tubulin inhibitor effectively inhibits NF-κB signaling to regulate immune responses and inflammatory mechanisms, and increases the expression of tight junction proteins (Claudin-1, ZO-1, Occludin) to strengthen a skin barrier function. In addition, the tubulin inhibitor inhibits the melanin production by inhibiting tyrosinase activity to exhibit a whitening effect.

Therefore, the tubulin inhibitor of the present disclosure can be utilized as an active ingredient that simultaneously exhibits activities, such as skin moisturization, whitening, wrinkle alleviation, acne relief, skin barrier strengthening, or skin keratinocyte differentiation, along with the prevention, treatment, or alleviation of skin diseases.

### [Description of drawings]

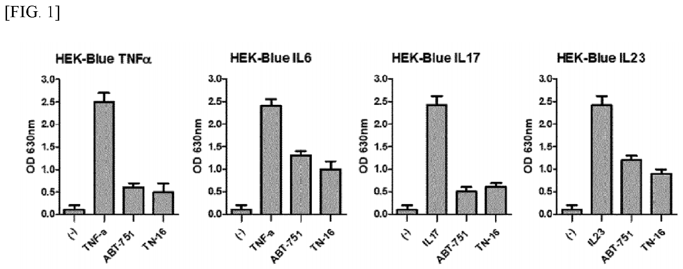

FIG. 1 relates to an effect of ABT-751 or TN-16 on inhibiting inflammatory cytokine (TNF-α, IL-6, IL-17, IL-23) signaling. As a result of measuring the degree of inhibition of signaling of each cytokine at OD630mm, it is shown that when treated with ABT-751 or TN-16, inflammatory cytokine signaling is significantly reduced compared to a control group.

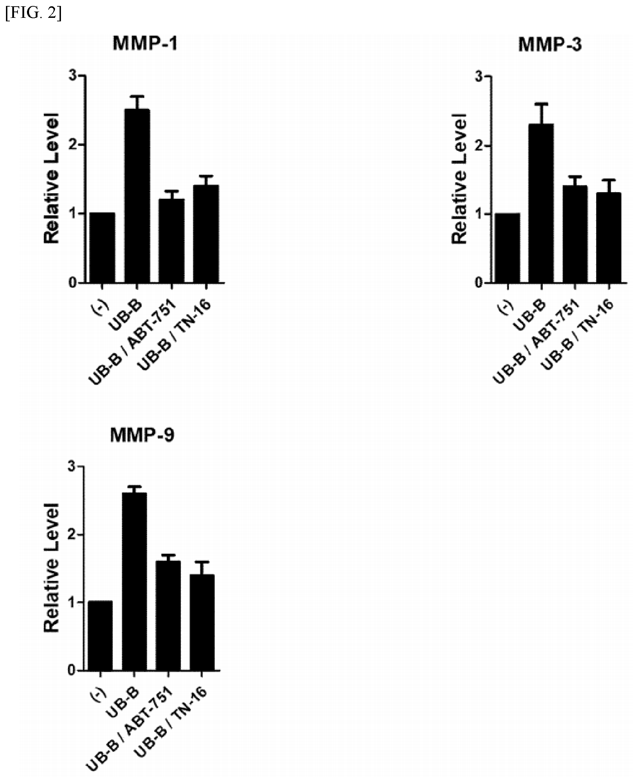

FIG. 2 relates to an effect of ABT-751 or TN-16 on inhibiting the expression of MMP-1, MMP-3, and MMP-9 induced by UV irradiation. As a result of measurement using RT-qPCR, it is shown that the MMP protein expression level in an ABT-751 or TN-16 treated group decreased to 50% or less compared to a control group.

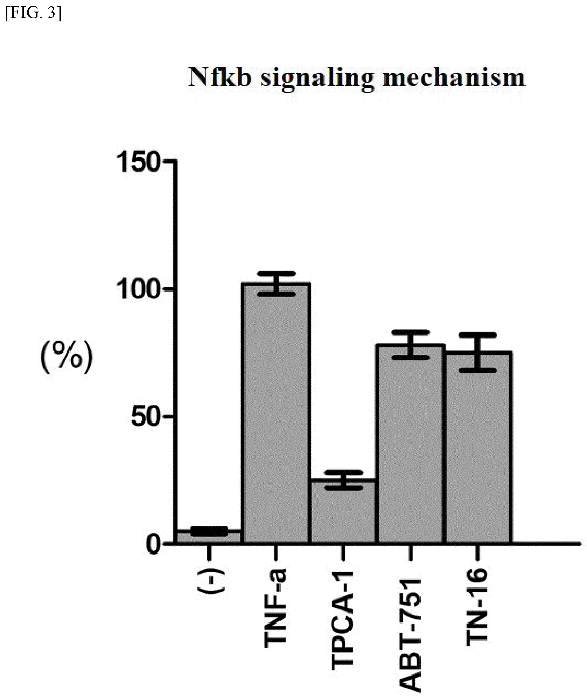

FIG. 3 relates to an effect of ABT-751 or TN-16 on inhibiting NF-κB signaling. As a result measured after treating a drug to cells with a lucifer luminescent gene inserted into a NF-κB promoter, ABT-751 or TN-16 exhibits a NF-κB signaling inhibitory effect similar to a control group and TPCA1, known as an NF-κB inhibitor.

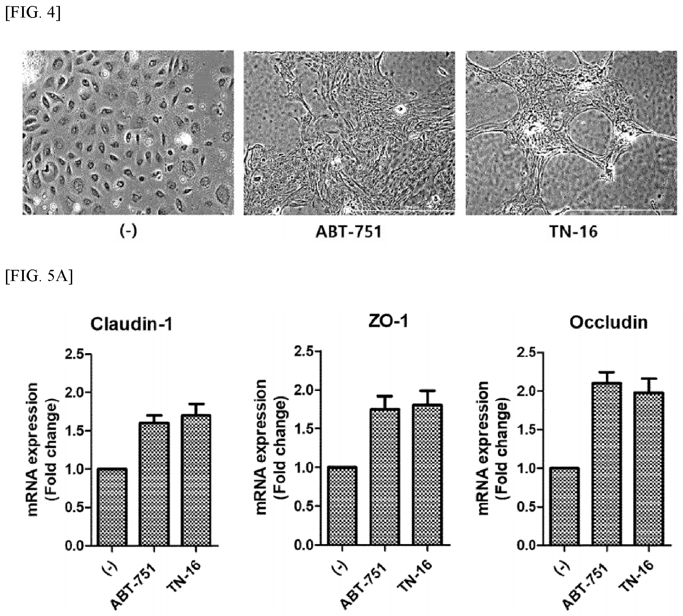

FIG. 4 relates to an effect of ABT-751 or TN-16 on increasing the differentiation degree of normal human epidermal keratinocyte (NHEK). A result of observing cells treated with a drug for 5 days using a phase-contrast microscope, it is shown that the differentiation degree of skin cells in the ABT-751 or TN-16 treated group is significantly increased compared to the control group.

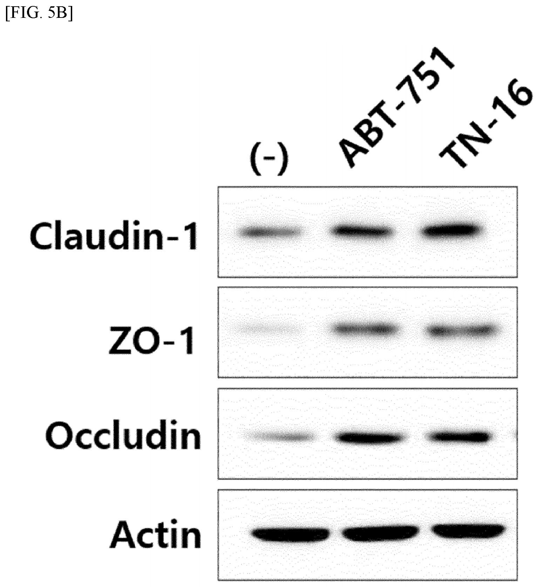

FIGS. 5A and 5B relate to an effect of ABT-751 or TN-16 on increasing the expression of tight junction proteins (Claudin-1, ZO-1, Occludin). As a result of RT-qPCR and Western Blot analysis, it is shown that the expression levels of Claudin-1, ZO-1, and Occludin are increased in the ABT-751 or TN-16 treated group compared to the control group.

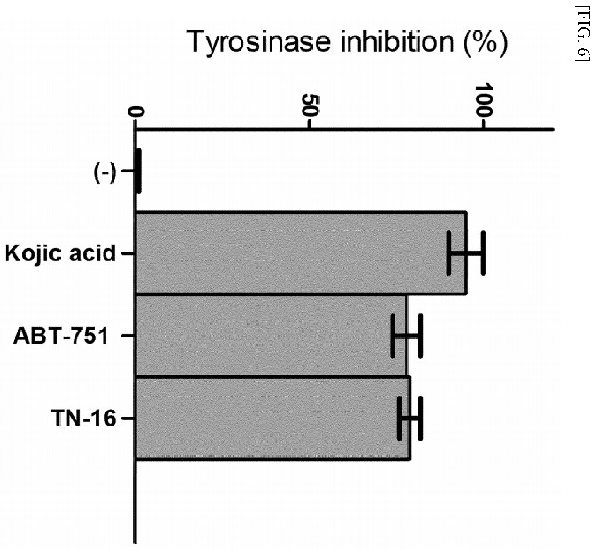

FIG. 6 relates to a whitening effect of ABT-751 or TN-16 on reducing melanin production by inhibiting Tyrosinase activity. As a result of a tyrosinase activity inhibition experiment, ABT-751 or TN-16 shows an effect of inhibiting melanin production.

### [Best Mode]

Hereinafter, the present disclosure will be described in detail.

The present disclosure provides a pharmaceutical composition for preventing or treating skin diseases including a compound represented by the following Chemical Formula I or II or a pharmaceutically acceptable salt thereof as an active ingredient.

In addition, the present disclosure provides a method for preventing or treating skin diseases including administering, to a subject in need thereof, a tubulin inhibitor which is a compound represented by the following Chemical Formula I or II or a pharmaceutically acceptable salt thereof, in a therapeutically effective amount.

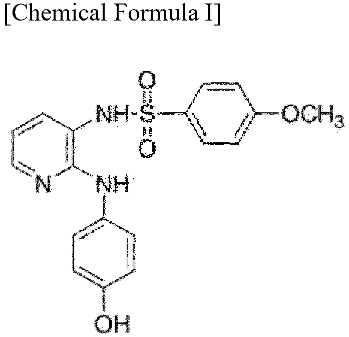

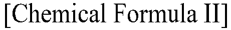

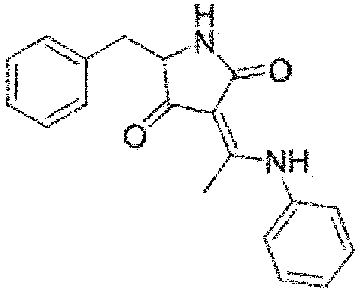

The tubulin inhibitor refers to a drug group that regulates or inhibits the function of tubulin protein as a major component of microtubules. The microtubule is a part of the cytoskeleton that plays an important role in cells and is an essential structure for processes such as cell division, cell migration, and intracellular transport.

In the present disclosure, unlike these previous functions, the tubulin inhibitor inhibits the signaling of inflammatory cytokines (TNF-α, IL-6, IL-17, IL-23) in epidermal cells to reduce inflammatory responses and inhibits the expression of Matrix Metalloproteinases (MMP-1, MMP-3, MMP-9) caused by ultraviolet rays (UV) to reduce collagen degradation and photoaging. In addition, the tubulin inhibitor effectively inhibits NF-κB signaling to regulate immune responses and inflammatory mechanisms, and increases the expression of tight junction proteins (Claudin-1, ZO-1, Occludin) to strengthen a skin barrier function. In addition, the tubulin inhibitor inhibits the melanin production by inhibiting tyrosinase activity to exhibit a whitening effect.

In the present disclosure, the tubulin inhibitor is ABT-751 represented by Chemical Formula I below, or TN-16 represented by Chemical Formula II below.

The compound I is a compound with the CAS registry number 141430-65-1, N-[2-[(4-Hydroxyphenyl)amino]pyridin-3-yl]-4-methoxybenzenesulfonamide (ABT-751).

ABT-751 is a compound targeting microtubules, and the drug has been reported to exhibit an activity of stopping the growth of cancer cells by inhibiting cell division in specific cancer cells.

In addition, the compound II is a compound with the CAS registry number 33016-12-5, 3-[1-(phenylamino)ethylidene]-5-(phenylmethyl)-2,4-pyrrolidinedione (TN-16).

The TN-16 is a novel microtubule inhibitor with antitumor activity, and is synthesized by modifying tenuazonic acid (3-acetyl-5-sec-butyltetramic acid), which is a natural antibiotic isolated and characterized from a culture solution of Alternaria tenuis. In 1967, the TN-16 was discovered to have potent anticancer efficacy, but subsequent studies also failed to find a more potent drug. In addition, in 1983, it was found that the mechanism of action of TN-16 inhibited microtubule formation and acted by binding to a sensitive site.

The tubulin inhibitor of the present disclosure is a concept including all pharmaceutically acceptable salts, isomers, or derivatives thereof, and analogs exhibiting the same or similar effects.

In the present disclosure, the pharmaceutically acceptable salts refer to salts commonly used in the pharmaceutical industry. For example, the pharmaceutically acceptable salts may include inorganic ion salts prepared with calcium, potassium, sodium, magnesium, etc.; inorganic acid salts prepared with hydrochloric acid, nitric acid, phosphoric acid, bromic acid, iodic acid, perchloric acid, sulfuric acid, etc.; organic acid salts prepared with acetic acid, trifluoroacetic acid, citric acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carboxylic acid, vanillic acid, hydroiodic acid, etc.; sulfonic acid salts prepared with methanesulfonic acid, ethanesulfonic acid, benzenesulfonic acid, p-toluenesulfonic acid, naphthalenesulfonic acid, etc.; amino acid salts made from glycine, arginine, lysine, etc.; and amine salts prepared with trimethylamine, triethylamine, ammonia, pyridine, picoline, etc., but are not limited thereto.

The tubulin inhibitor may be derived from an extract, produced biologically, or synthesized chemically, and is not limited in its origin and preparation method.

In the present disclosure, the tubulin inhibitor may exhibit the activity that upregulates cell tight junctions.

In the present disclosure, the skin diseases correspond to a group of diseases caused by structural and functional abnormalities of the skin, and mean a concept of encompassing abnormal conditions occurring in the skin that may be caused by various causes, such as inflammation, infection, allergy, and immune abnormality.

In the present disclosure, the skin disease may be a skin disease or skin injury caused by a decrease in the expression of claudin 1, Zonula Occludens-1 (ZO-1), or Occludin protein.

In the present disclosure, the skin disease or skin injury may be at least one selected from the group consisting of, for example, psoriasis, thermal skin injury, acne, allergic dermatitis, contact dermatitis, exfoliative dermatitis, seborrheic dermatitis, seborrheic scalpitis, lichen planus, rosacea, pigmentary disorder, hypermelanosis, erythema, wounds, ulcers, decubitis, lupus, skin wrinkle-related diseases, and skin diseases caused by photodamage, and the type thereof is not limited.

The skin wrinkle-related disease may include elastic fibrosis, thinning of the skin, skin atrophy, reduction of collagen fibers and elastic fibers, loss of skin elasticity, dryness, wrinkle formation, or premature skin aging.

In addition, the skin disease caused by the photodamage may include lentigines, freckles, hypopigmentation, hyperpigmentation, acute or chronic photodamage caused by UV radiation, or photosensitization.

In addition, the skin diseases of the present disclosure may include squamous cell carcinoma, basal cell carcinoma, benign epithelial tumor, and radiation dermatitis.

In addition, the skin diseases of the present disclosure may include panniculitis, calluses, vitiligo, urticaria, folliculitis, sebaceous keratosis pilaris, eczema, corns, melasma, rashes, athlete's foot, spots, stretch marks, freckles, prickly heat, dry skin, chilblains, boils, pockmarks, scalpitis or psoriatic arthritis.

The skin disease of the present disclosure may be, for example, a skin injury including psoriasis.

The histological characteristics of psoriatic lesions show a thickened epidermis due to abnormal keratinocyte proliferation and differentiation, as well as skin infiltration and co-localization of T lymphocytes and dendritic cells. The cause of psoriasis is defined primarily by the immune response of helper T cells, Th1 and Th17 cells, depending on genetic and protein analysis results. Accordingly, there are observed not only an increase in inflammatory cytokines directly related to skin diseases, such as interleukins IL-1α and IL-6 including tumor necrosis factor TNF-α, but also an increase in IL-17α and IL-23, which are involved in the immune response of Th17 cells. An increase in such inflammatory cytokines of TNF-α, IL-1α, IL-6, IL-17α, and IL-23 may be defined as the inflammatory signaling profile of psoriasis, and accordingly, drugs capable of reducing these cytokines may be used as therapeutic agents for psoriasis.

In addition, the present disclosure may further include a compound selected from the group consisting of calcium, colchicine, tapinarof, fingolimod, and tofacitinib, in addition to the tubulin inhibitor.

The compound selected from the group consisting of calcium, colchicine, tapinarof, fingolimod, and tofacitinib may be used in combination or mixed with the tubulin inhibitor, and may be simultaneously or sequentially administered.

The calcium, colchicine, tapinarof, fingolimod, or tofacitinib corresponds to a concept that includes all of their salts, isomers, or derivatives, and analogs that exhibit the same or similar effects.

In the present disclosure, the tubulin inhibitor may be provided as an active ingredient in the pharmaceutical composition for preventing or treating skin diseases.

The term "prevention" in the present disclosure may include inhibiting the occurrence of diseases.

The term "treatment" in the present disclosure includes inhibition, alleviation, or elimination of the development of diseases.

In the present disclosure, the term "including as the active ingredient" means that the tubulin inhibitor of the present disclosure is added so as to exhibit the effects mentioned in the present disclosure, and includes formulation in various forms by adding various ingredients as accessory ingredients for drug delivery, stabilization, etc.

The pharmaceutical composition may include the tubulin inhibitor in a pharmaceutically effective amount.

In the present disclosure, the term "pharmaceutically effective amount" means an amount sufficient to achieve the efficacy or activity of the active ingredient.

The pharmaceutical composition may further include a pharmaceutically acceptable diluent or carrier. The diluent may be lactose, corn starch, soybean oil, microcrystalline cellulose, mannitol, or a combination thereof. The carrier may be an excipient, a disintegrant, a binder, a lubricant, or a combination thereof. The excipient may be microcrystalline cellulose, lactose, low-substituted hydroxycellulose, or a combination thereof. The disintegrant may be calcium carboxymethylcellulose, sodium starch glycolate, calcium dihydrogen phosphate anhydrous, or a combination thereof. The binder may be polyvinylpyrrolidone, low-substituted hydroxypropyl cellulose, hydroxypropyl cellulose, or a combination thereof. The lubricant may be magnesium stearate, silicon dioxide, talc, or a combination thereof.

The pharmaceutical composition may be prepared into an oral or parenteral administration formulation. The oral formulations may include granules, powders, liquids, tablets, capsules, dry syrups, or combinations thereof. The parenteral formulations may include injections, ointments, aerosols, sprays, patches, etc., and the formulations thereof are not limited.

The dose of the pharmaceutical composition according to an embodiment may be administered to mammals for 1 day in an amount of, for example, about 0.0001 to 10,000 mg/kg, 0.0001 to 5,000 mg/kg, 0.0001 to 1,000 mg/kg, 0.0001 to 900 mg/kg, 0.0001 to 800 mg/kg, 0.0001 to 700 mg/kg, 0.0001 to 600 mg/kg, 0.0001 to 500 mg/kg, 0.0001 to 400 mg/kg, 0.0001 to 300 mg/kg, 0.0001 to 200 mg/kg, 1 to 1,000 mg/kg, 1 to 900 mg/kg, 1 to 800 mg/kg, 1 to 700 mg/kg, 1 to 600 mg/kg, 1 to 500 mg/kg, 1 to 450 mg/kg, 1 to 400 mg/kg, 1 to 350 mg/kg, 1 to 300 mg/kg, 1 to 250 mg/kg, 10 to 1,000 mg/kg, 10 to 900 mg/kg, 10 to 800 mg/kg, 10 to 700 mg/kg, 10 to 600 mg/kg, 10 to 500 mg/kg, 10 to 450 mg/kg, 10 to 400 mg/kg, 10 to 350 mg/kg, 10 to 300 mg/kg, 10 to 250 mg/kg, 100 to 1,000 mg/kg, 100 to 900 mg/kg, 100 to 800 mg/kg, 100 to 700 mg/kg, 100 to 600 mg/kg, 100 to 500 mg/kg, 100 to 450 mg/kg, 100 to 400 mg/kg, 100 to 350 mg/kg, 100 to 300 mg/kg, 100 to 250 mg/kg, 200 to 1,000 mg/kg, 200 to 900 mg/kg, 200 to 800 mg/kg, 200 to 700 mg/kg, 200 to 600 mg/kg, 200 to 500 mg/kg, 200 to 450 mg/kg, 200 to 400 mg/kg, 200 to 350 mg/kg, 200 to 300 mg/kg, or 200 to 250 mg/kg, but is not limited thereto. The frequency of administration of the pharmaceutical composition of the present disclosure is not particularly limited, but the pharmaceutical composition may be administered once a day or several times a day by dividing the dose. The dose is not limited to the scope of the present disclosure in any aspect.

The subject to be administered with the pharmaceutical composition provided in the present disclosure may be mammals, including human, dog, cat, horse, cow, pig, goat, rabbit, mouse, rat, etc., or cells, tissues, or culture solutions thereof isolated therefrom. In an example, the subject may be a subject (mammals such as human) or cells, tissues, or culture solutions thereof isolated therefrom in need of prevention, alleviation, and/or treatment of skin diseases or having inflammatory skin diseases as described above.

The pharmaceutical composition according to an embodiment may include the tubulin inhibitor in an amount of 1 to 80 wt%, 5 to 80 wt%, 5 to 75 wt%, 5 to 70 wt%, 5 to 65 wt%, 50 to 70 wt%, 55 to 65 wt%, 60 to 65 wt%, 10 to 60 wt%, 15 to 60 wt%, 20 to 60 wt%, 1 to 50 wt%, 5 to 50 wt%, 10 to 50 wt%, 15 to 50 wt%, 20 to 50 wt%, 1 to 40 wt%, 5 to 40 wt%, 10 to 40 wt%, 15 to 40 wt%, 20 to 40 wt%, 1 to 30 wt%, 5 to 30 wt%, 10 to 30 wt%, 15 to 30 wt%, 20 to 30 wt%, 1 to 25 wt%, 5 to 25 wt%, 10 to 25 wt%, 15 to 25 wt%, 20 to 25 wt%, or 23 to 25 wt%.

Alternatively, the composition may be a skin quasi-drug composition.

The skin quasi-drug may be cream, gel, ointment, skin emulsifier, skin suspension, transdermal patch, drug-containing bandage, lotion, spray, or a combination thereof, but is not limited thereto.

In the present disclosure, the skin quasi-drug may be appropriately mixed with ingredients commonly used in skin quasi-drugs such as cosmetics or medicines, such as aqueous ingredients, oily ingredients, powder ingredients, alcohols, moisturizers, thickeners, ultraviolet absorbers, whitening agents, preservatives, antioxidants, surfactants, fragrances, colorants, various skin nutrients, metal ion sequestrants, sugars, or combinations thereof, as needed.

In addition, the present disclosure provides a quasi-drug composition for preventing or alleviating skin diseases including, as an active ingredient, a tubulin inhibitor which is a compound represented by Chemical Formula I or II or a pharmaceutically acceptable salt thereof.

In the present disclosure, the quasi-drug refers to all products other than drugs related to the treatment or prevention of diseases.

In the present disclosure, the quasi-drug refers to all products other than drugs related to the treatment or prevention of diseases. The quasi-drug means products that have a milder effect than drugs among products used for the purpose of diagnosing, treating, alleviating, reducing, managing or preventing diseases of humans or animals. For example, according to the Pharmaceutical Affairs Act, the quasi-drugs are products excluding products used for the purpose of medicines, and may include fiber and rubber products used for the treatment or prevention of diseases of humans or animals, products that have a mild or no direct effect on the human body and are not instruments or machines or similar thereto, sterilizers and insecticides for preventing infectious diseases, etc., but are not limited thereto.

The type or formulation of the quasi-drug composition of the present disclosure is not particularly limited, but may preferably be a disinfectant, a shower foam, a gargle, a wet tissue, a detergent soap, a hand wash, a humidifier filler, a mask, an ointment, a filter filler, or the like.

When the composition of the present disclosure is included in the quasi-drug for the purpose of skin moisturization, the composition may be used as it is or used in combination with other quasi-drug ingredients, and may be used appropriately according to conventional methods. The mixing amount of the active ingredient may be appropriately determined according to the purpose of use, and the quasi-drug composition according to the present disclosure may contain the tubulin inhibitor in an amount of 0.01 to 20 wt% based on the total weight of the composition.

In addition, the present disclosure provides a cosmetic composition for preventing or alleviating skin diseases including, as an active ingredient, a tubulin inhibitor which is a compound represented by Chemical Formula I or II or a pharmaceutically acceptable salt thereof.

The term "alleviation" in the present disclosure may mean all actions that at least reduce parameters associated with alleviation or treatment of conditions, for example, the degree of symptoms.

The tubulin inhibitor may be included in an amount of 0.0001 to 80 wt%, 0.01 to 70 wt%, 0.01 to 60 wt%, 0.01 to 50 wt%, 0.01 to 40 wt%, 0.01 to 30 wt%, 0.1 to 70 wt%, 0.1 to 60 wt%, 0.1 to 50 wt%, 0.1 to 40 wt%, 0.1 to 30 wt%, 0.5 to 70 wt%, 0.5 to 60 wt%, 0.5 to 50 wt%, 0.5 to 40 wt%, 0.5 to 30 wt%, 1 to 70 wt%, 1 to 60 wt%, 1 to 50 wt%, 1 to 40 wt%, or 1 to 30%, for example, 30 wt% based on the total weight of the cosmetic composition, but is not limited thereto.

The cosmetic composition is not particularly limited to a specific formulation, and the formulation may be appropriately selected depending on the purpose. The cosmetic composition may have, for example, a solubilized formulation, an emulsified formulation, or a dispersed formulation. The cosmetic composition may have a cosmetic formulation of a skin toner, a nourishing toner, a massage cream, a nourishing cream, an essence, a pack, a gel, an ampoule, or a skin adhesive type, but is not limited thereto.

The cosmetic composition may further include ingredients commonly used in the cosmetic composition in addition to the active ingredient described in the present disclosure, and may include, for example, conventional adjuvants and carriers, such as antioxidants, stabilizers, solubilizers, surfactants, dispersants, emulsifiers, preservatives, vitamins, pigments, and fragrances.

In addition, the present disclosure provides a cosmetic composition including, as an active ingredient, a tubulin inhibitor which is a compound represented by Chemical Formula I or II or a pharmaceutically acceptable salt thereof, for skin moisturization, whitening, wrinkle alleviation, acne relief, skin barrier strengthening, or skin keratinocyte differentiation.

In the present disclosure, the term "skin moisturization" may mean all actions that maintain skin moisture or prevent moisture loss.

In the present disclosure, the term "whitening" may mean improving the brightness of skin tone or alleviating pigmentation by inhibiting melanin production.

In the present disclosure, the term "wrinkle alleviation" may mean increasing skin elasticity, reducing the depth of wrinkles, or increasing the smoothness of the skin surface.

In the present disclosure, the term "acne relief" may mean inhibiting inflammation, redness, or excessive sebum production of acne, or promoting healing of acne lesions.

In the present disclosure, the term "skin barrier strengthening" may mean all actions that enhance the functions of the skin barrier, which is located at the outermost part of the skin to prevent moisture and nutrient loss.

In the present disclosure, the term "skin keratinocyte differentiation" may mean promoting a process of maturing keratinocytes to form the stratum corneum, or strengthening the function of the skin barrier.

Yet another aspect of the present disclosure provides a method for preventing, alleviating, or treating skin diseases, including administering an effective amount of the tubulin inhibitor to a subject in need thereof.

The term "effective amount" means an amount effective to exhibit the effects mentioned above.

The subject may be mammals, for example, human, cow, horse, pig, dog, sheep, goat, or cat. The subject may be a subject requiring the effect of preventing, alleviating, or treating skin diseases.

In the present disclosure, the term "administering" is used interchangeably with "introducing" or "implanting", and may mean placement of a composition according to an embodiment into a subject by a method or route that causes at least partial localization of the composition according to an embodiment to a desired site.

The administration may be performed by methods known in the art. The administration method, including the route of administration and the number of administrations, may be appropriately selected by those skilled in the art. For example, the administration may be directly performed to the subject by any means through routes, such as intravenous, intramuscular, oral, transdermal, mucosal, intranasal, intraoral, intratracheal or subcutaneous administration. The administration may be performed systemically or locally. The administration may include application to the skin.

In the present disclosure, the composition according to one specific example may be administered per subject a day in an amount of 0.1 mg to 1,000 mg, for example, 0.1 mg to 500 mg, 0.1 mg to 100 mg, 0.1 mg to 50 mg, 0.1 mg to 25 mg, 1 mg to 1,000 mg, 1 mg to 500 mg, 1 mg to 100 mg, 1 mg to 50 mg, 1 mg to 25 mg, 5 mg to 1,000 mg, 5 mg to 500 mg, 5 mg to 100 mg, 5 mg to 50 mg, 5 mg to 25 mg, 10 mg to 1,000 mg, 10 mg to 500 mg, 10 mg to 100 mg, 10 mg to 50 mg, or 10 mg to 25 mg. However, the dose may be variously prescribed by factors, such as a formulation method, an administration method, the age, weight, sex, and a pathological condition of a patient, diet, an administration time, an administration route, an excretion rate, and response susceptibility, and those skilled in the art may appropriately adjust the dose by considering these factors. The number of administrations may be once a day or twice or more within the range of clinically acceptable side effects, and the administration site may be one or two or more sites, and the total number of administration days may be from 1 to 30 days during once treatment, every day or at intervals of 2 to 5 days. If necessary, the same treatment may be repeated after an appropriate period of time. Even for animals other than humans, the same dose per kg as for humans may be administered, or the dose may be administered in an amount converted the above-described dose by a volume ratio (e.g., average value) of organs (heart, etc.) between target animal and human.

Yet another aspect provides a use of the tubulin inhibitor for use in preparing a composition for preventing, alleviating, or treating of skin diseases.

Yet another aspect provides a use of the tubulin inhibitor for use in preparing a drug for preventing, alleviating, or treating skin diseases.

Yet another aspect provides a food composition including a tubulin inhibitor of the present disclosure as an active ingredient for preventing or alleviating skin diseases.

The food composition according to an embodiment of the present disclosure may be liquid or solid formulations, and may be tablets, capsules, soft capsules, pills, granules, beverages (drinks), diet bars, chocolate, caramel formulations, or confectionery formulations, and the formulations are not particularly limited. The food composition of the present disclosure may appropriately contain excipients, sugars, flavorings, pigments, fats, proteins, etc. as needed, in addition to the active ingredient.

Duplicated contents will be omitted in consideration of the complexity of the present specification, and terms not defined otherwise herein have the meanings commonly used in the art to which the present disclosure pertains.

### [Modes]

Hereinafter, the present disclosure will be described in detail by Examples. However, the following Examples are just illustrative of the present disclosure, and the contents of the present disclosure are not limited to the following Examples.

### [Example 1] Mechanism of inhibition of inflammatory cytokines by tubulin inhibitors

HEK293 cell lines (Promega) having genes with a colorimetric function inserted into respective signaling promoters of TNF-α, IL-2, IL-4/13, IL-6, IL-12, IL-17, and IL-23 were purchased. The degree of signaling in each cell line may be measured based on the degree to be developed in blue when each signaling was induced.

Each HEK-Blue cell line was treated with a drug of 1 µM ABT-751 or 1 µM TN-16, and then after 6 hours, a signaling inducer was treated to each cell line. The degree of signaling of each induced inflammatory response was measured at OD630mm, and the results were shown in FIG. 1.

As may be seen in FIG. 1, when treated with the drug of 1 µM ABT-751 or 1 µM TN-16, a reduction in the signaling of inflammatory cytokines TNF-α and IL-6, IL-17, and IL-23 was confirmed.

It was confirmed the effect of the drug ABT-751 or TN-16 on reducing TNF-α and IL-6, IL-17, and IL-23 cytokines, which were skin inflammation signal profiles, from an inflammation site.

### [Example 2] Confirmation of collagen degradation inhibitory effect of tubulin inhibitor

Photoaging referred to a skin aging phenomenon that occurred when the skin was exposed to UV of the sun. The photoaging was a form of aging distinguished from biological aging (or senescence) due to a gradual decline in body functions and acted generally as the main cause of skin wrinkles, dryness, fibroelastosis, pigmentation, or the like.

The sun emitted two types of UV such as UV-A and UV-B, and the wavelengths were 320 to 400 nm for UV-A and 290 to 320 nm for UV-B. Among tissues damaged by the UV, collagen was a representative example. The collagen was the main ingredient of connective tissue and was mainly present in bones and skin and broadly classified into five types. Type 1 was the most abundant collagen found within the skin connective tissue, and Type 3 was the second abundant collagen.

Matrix Metalloproteinase (MMP) was a protease that degraded collagen, etc. as a major component of human connective tissue, and the expression level thereof was increased by UV. Accordingly, when the skin was exposed to UV, the expression of these Matrix Metalloproteinases (MMPs) increased to destroy collagen in the dermal layer of the skin and cause photoaging such as skin wrinkles and pigmentation.

The human NHEK cells were applied with ABT-751 or TN-16, respectively, and irradiated with UV light after 30 minutes to induce photoaging. For comparison and verification of ABT-751 or TN-16, an untreated negative control group and a positive control group irradiated only with UV-A were reflected in the experiment.

RNA was extracted from skin cells after application and UV irradiation experiment completion. After reverse transcription of the extracted RNA into cDNA, the amounts of MMP-1, MMP-3, and MMP-9 present in skin tissue were measured using a real-time PCR machine by a quantitative PCR (qPCR) method.

Specifically, a photoaging inhibitory effect of ABT-751 or TN-16 was measured through changes in the expression levels of Matrix Metalloproteinase (MMP) proteins as described above. That is, the expression levels of the MMP proteins were measured in each of a control group irradiated with only UV and an experimental group irradiated with UV after applying ABT-751 or TN-16, respectively, to measure the degree of inhibiting the expression levels of MMP proteins by applying ABT-751 or TN-16, respectively.

The results were shown in FIG. 2.

MMP-1 and MMP-9 were enzymes that promoted the degradation of Type 1 and Type 3 collagens, which were mainly expressed in skin tissue, and MMP-3 was a regulatory enzyme that degraded Type 1 collagen and regulated changes in the expression level of MMP-1, which were MMP proteins that had a very important effect on photoaging.

As may be seen in FIG. 2, when ABT-751 or TN-16 was applied to the skin, respectively, it was confirmed that the expression levels of MMP-1, MMP-3, and MMP-9 were mostly reduced to 50% or less compared to the control group irradiated with only UV. As such, it was verified that the expression levels of Matrix Metalloproteinases (MMP) proteins were inhibited by the application of ABT-751 or TN-16, which was a result indicating that ABT-751 or TN-16 may be effective for wrinkle-related diseases caused by collagen reduction or skin diseases caused by photodamage.

### [Example 3] Confirmation of Nfkb inhibitory effect of tubulin inhibitor

NF-κB, as a DNA transcription factor, was present in the cytosol and present in a form conjugated with IκB, which inhibited NF-κB. The NF-κB was a representative pro-inflammatory cytokine that was involved in innate and acquired immune responses and shown in many inflammatory diseases.

The NF-κB mechanism was a protein family involved in inflammatory response regulation, immune modulation, apoptosis, cell proliferation, epithelial differentiation, etc. and regulated the expression of various genes and formed a central axis of the intracellular signaling system.

In the experiment, biochemical changes in cells were confirmed through treatment of HEK293 cell lines with ABT-751 or TN-16 to test the inflammatory response.

A human embryonic kidney cell line (HEK) was inserted with a lucifer luminescent gene having an NFkb promoter using a Fugene4K reagent (Promega), and then treated with TNF-α after 6 hours to induce inflammation and NfKb signaling. After 18 hours, ABT-751 or TN16 was treated at a concentration of 1.0 µM.

After 24 hours of drug treatment, an Nfkb signaling mechanism inhibition effect according to treatment of each tubulin inhibitor was measured using a kit (Promega) for measuring the level of lucifer luminescence, and the results were shown in FIG. 3.

As shown in FIG. 3, it was confirmed that treatment with ABT-751 or TN-16 according to the present disclosure inhibited NF-kB signaling similarly to TPCA1, which was known as a control substance and an NF-kB signaling inhibitor.

### [Example 4] Confirmation of skin cell differentiation ability of tubulin inhibitor

After applying ABT-751 or TN-16 using a normal human epidermal keratinocyte (NHEK), the degree of skin cell differentiation was observed and compared with the control group.

The cells were treated with ABT-751 or TN-16 at a concentration of 1 µM, respectively, and then the degree of skin cell differentiation was observed daily using a phase-contrast microscope. The result in FIG. 4 showed cells on day 5 of differentiation taken by a phase-contrast microscope camera. It may be confirmed that the degree of differentiation significantly increases in skin cells treated with ABT-751 or TN16 compared to an untreated control group.

### [Example 5] Confirmation of tight junction-related efficacy of tubulin inhibitor

Tight junctions were important structures that formed close connections between epithelial and endothelial cells to regulate the movement of substances between cells and maintain a barrier function. This structure was mainly composed of proteins such as Claudin-1, ZO-1, and Occludin, and each played a specific role. Claudin-1 was a key membrane protein of tight junctions that regulated selective permeability, Occludin enhanced structural stability, and ZO-1 was a scaffold protein that connected the cytoskeleton with other proteins.

The expression of tight junction proteins Claudin-1, ZO-1, and Occludin was essential for maintaining a skin barrier function and strengthening defense against external stimuli. When the expression of these proteins decreased or their functions were impaired, various skin diseases such as atopic dermatitis, psoriasis, and dry skin may be developed or worsened. Therefore, therapeutic strategies that restored or regulated the expression of tight junction proteins have received attention as an important approach in the prevention and treatment of skin diseases.

To confirm whether the tubulin inhibitor of the present disclosure was involved in tight junctions, the expression levels of Claudin-1, ZO-1, and Occludin were measured using reverse transcription real-time polymerase chain reaction (RT-qPCR).

Intracellular RNA was extracted from NHEKs coated with 1 µM concentration of ABT-751 or TN-16, respectively, after 6 hours using an RNeasy mini kit (Qiagen). After reverse transcription of the extracted RNA into cDNA using an ImProm-II^{™} Reverse Transcription kit (Promega), the expression levels of Claudin-1, ZO-1, and Occludin present in skin cells were measured by a quantitative PCR (qPCR) method using the Real-Time PCR Detection System (Bio-Rad, CFX96). The results were shown in FIG. 5A.

In addition, intracellular proteins were extracted from NHEKs coated with 1 µM concentration of ABT-751 or TN-16, respectively, after 24 hours. The expression levels of Claudin-1, ZO-1, and Occludin proteins present in skin cells among the extracted proteins were measured using Western blot.

A portion of the collected tissue was homogenized using a tissue homogenizer (Daihan Science Co.), lysed using a cell lysis buffer (RIPA buffer, Invitrogen), and centrifuged using a centrifuge (Labogene), and the supernatant was transferred to a new tube to be used as a protein sample.

30 µg of protein samples was loaded onto a PAGE gel (Invitrogen) with a concentration gradient of 4% to 12%, and SDS-PAGE was performed using a running buffer (Invitrogen) and an electrophoresis device (Bio-Rad) to separate the proteins by molecular weight. The separated proteins were transferred to a PVDF thin film (Bio-Rad) and then blocked with a 5% blocking solution (Skim milk; BD).

The thin film was reacted with claudin-1, ZO-1, occludin antibody (Santa Cruz), or Actin antibody (Abcam) for 16 hours, washed, and then reacted again with a secondary antibody for 1 hour and washed.

The thin film was reacted with an ECL solution (Thermo Fisher), and then the reacted protein was detected using a luminescence measuring instrument (Fusion Solo, Vilber). The results were shown in FIG. 5B.

The results of FIGS. 5A and 5B showed that the expression levels of Claudin-1, ZO-1, and Occludin increased in skin cells treated with ABT-751 or TN16 compared to the control group, which indicated that the tubulin inhibitor ABT-751 or TN-16 of the present disclosure strengthened the tight junctions of the cells.

### [Example 6] Confirmation of whitening effect of tubulin inhibitor

Tyrosinase was an enzyme involved in the most important initial rate determining step in a melanin biosynthesis pathway in the human body, and many whitening ingredients had a mechanism of action that inhibited this enzyme. This test was a method for evaluating the degree of inhibiting tyrosinase activity in vitro.

The whitening effect of ABT-751 or TN-16 was confirmed by measuring the effect through an in vitro tyrosinase activity inhibition experiment during in vivo experiments. Kojic acid, known to inhibit the tyrosinase activity, was used as a comparative group.

Through the tyrosinase activity inhibition experiment, ABT-751 or TN-16 was treated to a concentration of 1.0 µM in a 2 mM L-tyrosine substrate and measured at 490 nm absorbance, and then added with tyrosinase and reacted at 37°C. After measuring the absorbance at 490 nm again, the absorbance value was calculated to determine the degree to inhibit the tyrosinase activity of ABT-751 or TN-16. The results were shown in FIG. 6.

As may be seen in FIG. 6, ABT-751 or TN-16, which was a tubulin inhibitor of the present disclosure, exhibited an effect of inhibiting melanin production.

## Claims

1. A pharmaceutical composition for preventing or treating skin diseases comprising, as an active ingredient, a tubulin inhibitor which is a compound represented by Chemical Formula I or II or a pharmaceutically acceptable salt thereof.

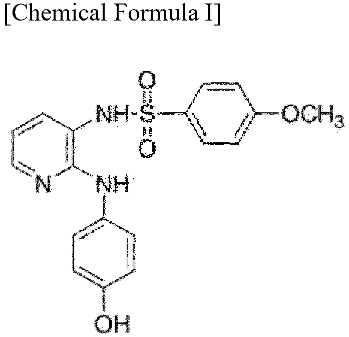

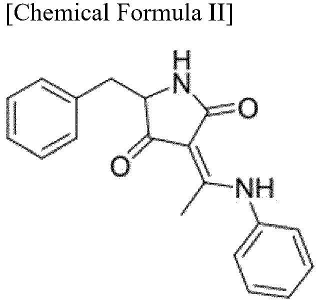

2. The pharmaceutical composition of claim 1, wherein the tubulin inhibitor upregulates cell tight junctions.

3. The pharmaceutical composition of claim 1, wherein the skin diseases are skin diseases caused by a decrease in the expression of claudin 1, Zonula Occludens-1 (ZO-1), or Occludin protein.

4. The pharmaceutical composition of claim 1, wherein the skin disease is at least one selected from the group consisting of psoriasis, thermal skin injury, acne, allergic dermatitis, contact dermatitis, exfoliative dermatitis, seborrheic dermatitis, seborrheic scalpitis, lichen planus, rosacea, pigmentary disorder, hypermelanosis, erythema, wounds, ulcers, decubitis, lupus, skin wrinkle-related diseases, and skin diseases caused by photodamage.

5. The pharmaceutical composition of claim 4, wherein the skin wrinkle-related disease is at least one selected from the group consisting of elastic fibrosis, thinning of the skin, skin atrophy, reduction of collagen fibers and elastic fibers, loss of skin elasticity, dryness, wrinkle formation, and premature skin aging.

6. The pharmaceutical composition of claim 4, wherein the skin disease caused by the photodamage is at least one selected from the group consisting of lentigines, freckles, hypopigmentation, hyperpigmentation, photodamage caused by acute or chronic UV radiation, and photosensitization.

7. The pharmaceutical composition of claim 1, wherein the skin disease is at least one selected from the group consisting of squamous cell carcinoma, basal cell carcinoma, benign epithelial tumor, and radiation dermatitis.

8. The pharmaceutical composition of claim 1, wherein the skin disease is at least one selected from the group consisting of panniculitis, calluses, vitiligo, urticaria, folliculitis, sebaceous keratosis pilaris, eczema, corns, melasma, rashes, athlete's foot, spots, stretch marks, freckles, prickly heat, dry skin, chilblains, boils, pockmarks, scalpitis and psoriatic arthritis.

9. A quasi-drug composition for alleviating skin diseases comprising, as an active ingredient, a tubulin inhibitor which is a compound represented by Chemical Formula I or II or a pharmaceutically acceptable salt thereof.

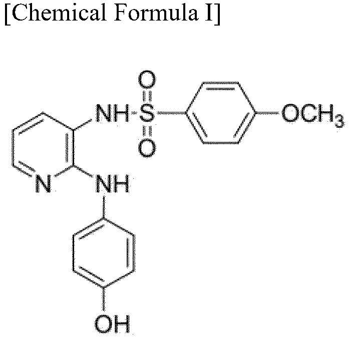

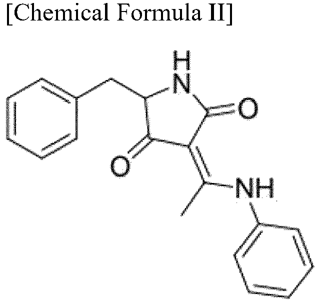

10. A cosmetic composition for preventing or alleviating skin diseases comprising, as an active ingredient, a tubulin inhibitor which is a compound represented by Chemical Formula I or II or a pharmaceutically acceptable salt thereof.

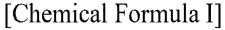

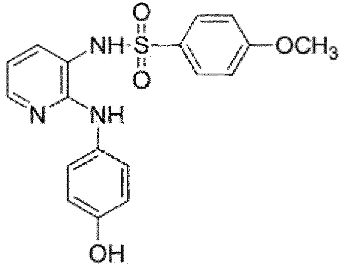

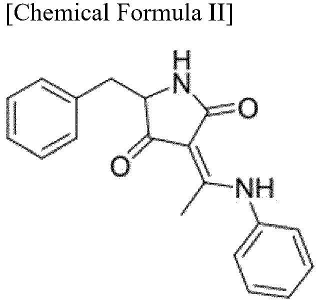

11. A cosmetic composition comprising, as an active ingredient, a tubulin inhibitor which is a compound represented by Chemical Formula I or II or a pharmaceutically acceptable salt thereof, for skin moisturization, whitening, wrinkle alleviation, acne relief, skin barrier strengthening, or skin keratinocyte differentiation.

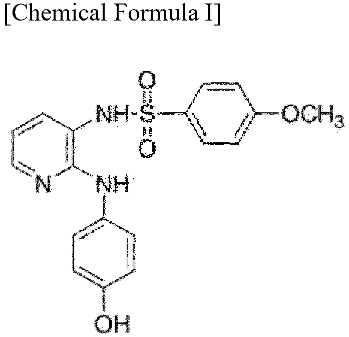

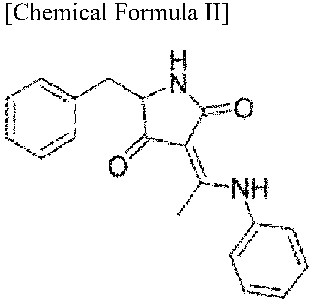

12. A method for preventing or treating skin diseases comprising administering, to a subject in need thereof, a tubulin inhibitor which is a compound represented by the following Chemical Formula I or II or a pharmaceutically acceptable salt thereof, in a therapeutically effective amount.
